(19) Europäisches Patentamt
European Patent Office
Office européen des brevets

(11) Veröffentlichungsnummer: **0 477 637 A1**

(12) **EUROPÄISCHE PATENTANMELDUNG**

(21) Anmeldenummer: **91115182.7**

(22) Anmeldetag: **09.09.91**

(51) Int. Cl.5: **C07D 239/60**, C07D 239/28, C07D 239/32, C07D 239/46, C07D 251/26, C07D 251/30, C07D 251/38, A01N 43/54, A01N 43/70

(30) Priorität: **22.09.90 DE 4030041**

(43) Veröffentlichungstag der Anmeldung: **01.04.92 Patentblatt 92/14**

(84) Benannte Vertragsstaaten: **BE CH DE DK ES FR GB IT LI NL**

(71) Anmelder: **BAYER AG**

**W-5090 Leverkusen 1 Bayerwerk(DE)**

(72) Erfinder: **Andree, Roland, Dr.**
**Schillerstrasse 17**
**W-4018 Langenfeld(DE)**
Erfinder: **Drewes, Mark Wilhelm, Dr.**
**Grünstrasse 30**
**W-4018 Langenfeld(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch Gladbach 2(DE)**
Erfinder: **Schmidt, Robert R., Dr.**
**Im Waldwinkel 110**
**W-5060 Bergisch Gladbach 2(DE)**

(54) **Bisazinylverbindungen.**

(57) Die Erfindung betrifft neue Bisazinylverbindungen der Formel (I),

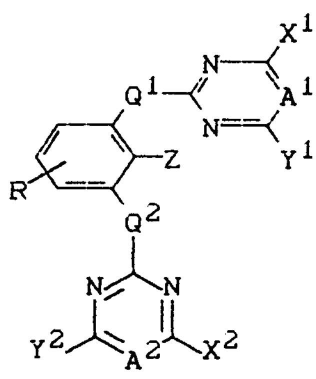

(I)

in welcher

$A^1$ und $A^2$ gleich oder verschieden sind und für Stickstoff oder eine C-X-Gruppierung stehen, wobei X für Wasserstoff, Halogen, Alkyl oder Alkoxy steht, und

$Q^1$ und $Q^2$ gleich oder verschieden sind und für Sauerstoff, Schwefel, NH oder N-Alkyl stehen,

(wobei die Substituenten R, $X^1$, $X^2$, $Y^1$, $Y^2$ und Z die in der Beschreibung angegebenen Bedeutung haben), Verfahren und neue Zwischenprodukte zu ihrer Herstellung sowie ihre Verwendung als Herbizide.

EP 0 477 637 A1

Die Erfindung betrifft neue Bisazinylverbindungen, mehrere Verfahren und neue Zwischenprodukte zu ihrer Herstellung sowie ihre Verwendung als Herbizide.

Es ist bereits bekannt, daß bestimmte Bisazinylverbindungen herbizide Eigenschaften aufweisen (vgl. EP-A 008192, EP-A 321846 und EP 336494). Verbindungen aus den genannten Publikationen haben jedoch bisher keine größere Bedeutung erlangt.

Es wurden nun die neuen Bisazinylverbindungen der allgemeinen Formel (I) gefunden,

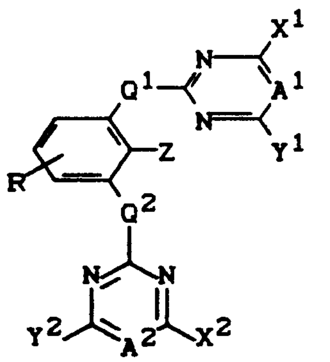

(I)

in welcher

$A^1$ und $A^2$ gleich oder verschieden sind und für Stickstoff oder eine C-X-Gruppierung stehen, wobei X für Wasserstoff, Halogen, Alkyl oder Alkoxy steht,

$Q^1$ und $Q^2$ gleich oder verschieden sind und für Sauerstoff, Schwefel, NH oder N-Alkyl stehen,

R für Wasserstoff, Amino, Hydroxy, Cyano, Nitro, Halogen, Alkyl, Halogenalkyl, Alkoxy, Halogenalkoxy, Alkylthio, Halogenalkylthio, Alkylamino, Dialkylamino, Alkylcarbonylamino, Alkoxycarbonylamino oder Alkylsulfonylamino steht,

$X^1$, $X^2$, $Y^1$ und $Y^2$ gleich oder verschieden sind und für Wasserstoff, Halogen, Alkyl, Halogenalkyl, Alkoxy, Halogenalkoxy, Alkylthio, Alkylamino, Dialkylamino oder für gegebenenfalls substituiertes Phenoxy stehen, und

Z für eine der nachstehenden Gruppierungen steht:

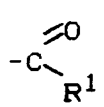 , 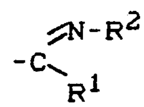 , 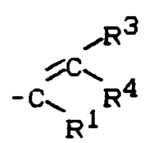 ,

wobei

$R^1$ für Wasserstoff, für gegebenenfalls substituiertes Alkyl oder für gegebenenfalls substituiertes Phenyl steht,

$R^2$ für Wasserstoff, Hydroxy, Amino oder für jeweils gegebenenfalls substituiertes Alkyl, Alkenyl, Alkinyl, Alkoxy, Alkenyloxy, Alkoxycarbonylalkoxy, Dialkylaminocarbonylalkoxy, Alkylamino, Dialkylamino, Alkylcarbonylamino, Alkoxycarbonylamino, Alkylsulfonylamino, Aryl, Aralkyl, Aryloxy, Aralkyloxy, Arylamino, Diarylamino, Aralkylamino, N-Alkyl-N-arylamino, Arylcarbonylamino, Heteroarylamino, Heteroarylcarbonylamino oder Arylsulfonylamino steht,

$R^3$ für Wasserstoff, Halogen, Cyano, Carboxy, Alkoxycarbonyl, Alkylcarbonylamino oder Dialkoxyphosphoryl steht, und

$R^4$ für Formyl, Cyano, Carboxy, Hydroxymethyl, Carbamoyl oder für jeweils gegebenenfalls substituiertes Alkoxycarbonyl, Cycloalkoxycarbonyl, Alkylthiocarbonyl, Alkylaminocarbonyl, Cycloalkylaminocarbonyl, Dialkylaminocarbonyl, Alkylaminocarbonylalkoxycarbonyl, Dialkylaminocarbonylalkoxycarbonyl, Arylaminocarbonylalkoxycarbonyl, N-Alkyl-N-arylaminocarbonylalkoxycarbonyl, Pyrrolidinylcarbonyl, Piperidinylcarbonyl, Morpholinylcarbonyl, Piperazinylcarbonyl, Aryloxycarbonyl, Aralkyloxycarbonyl, Heterocyclylalkoxycarbonyl, Arylthiocarbonyl, Aralkylthiocarbonyl, Arylaminocarbonyl, Aralkylaminocarbonyl, N-Alkyl-N-arylaminocarbonyl, Arylhydrazino-

carbonyl, Alkylhydrazinocarbonyl, Phthalimidoxycarbonyl oder $R^4$ zusammen mit $R^3$ für die Gruppierung $-CO-O-(CH_2)_n-$ steht, wobei n für die Zahlen 1 bis 4 steht.

Man erhält die neuen Bisazinylverbindungen der allgemeinen Formel (I), wenn man

(a) Hydroxyverbindungen der allgemeinen Formel (II),

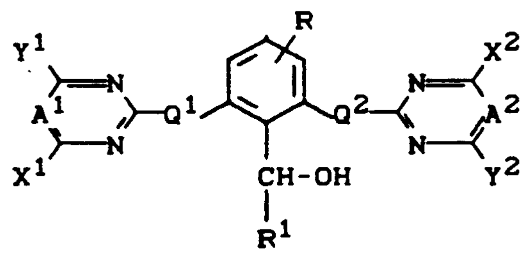

(II)

in welcher

$A^1$, $A^2$, $Q^1$, $Q^2$, R, $R^1$, $X^1$, $X^2$, $Y^1$ und $Y^2$ die oben angegebene Bedeutung haben,

mit Oxidationsmitteln ("Dehydrierungsmitteln") gegebenenfalls in Gegenwart von Verdünnungsmitteln umsetzt, oder wenn man

(b) Benzolderivate der allgemeinen Formel (III),

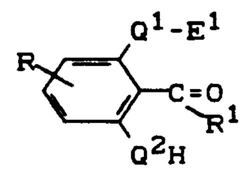

(III)

in welcher

$Q^1$, $Q^2$, R und $R^1$ die oben angegebene Bedeutung haben und

$E^1$ für Wasserstoff oder die Gruppierung

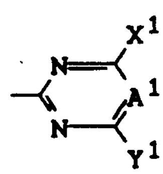

steht, wobei

$A^1$, $X^1$ und $Y^1$ die oben angegebene Bedeutung haben,

mit reaktionsfähigen Azinen der allgemeinen Formel (IVa),

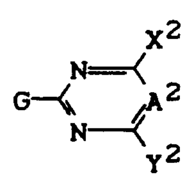

(IVa)

und gegebenenfalls auch mit reaktionsfähigen Azinen der allgemeinen Formel (IVb),

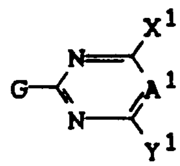

(IVb)

wobei

$A^1$, $A^2$, $X^1$, $X^2$, $Y^1$ und $Y^2$ die oben angegebene Bedeutung haben und

G für eine nucleophile Abgangsgruppe steht,

gegebenenfalls in Gegenwart eines Säureakzeptors und gegebenenfalls in Gegenwart eines Verdünnungsmittels umsetzt, oder wenn man

(c) Carbonylverbindungen der allgemeinen Formel (Ia),

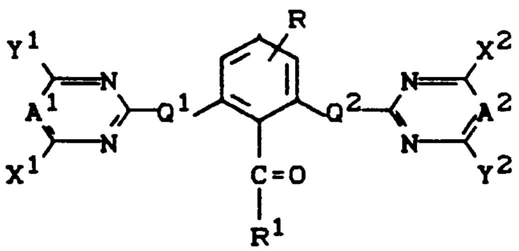

(Ia)

in welcher

$A^1$, $A^2$, $Q^1$, $Q^2$, R, $R^1$, $X^1$, $X^2$, $Y^1$ und $Y^2$ die oben angegebene Bedeutung haben,

mit Aminoverbindungen der allgemeinen Formel (V),

$H_2N\text{-}R^2$ (V)

in welcher

$R^2$ die oben angegebene Bedeutung hat,

oder mit Methylenverbindungen der allgemeinen Formel (VI),

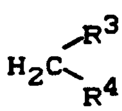

(VI)

in welcher

$R^3$ und $R^4$ die oben angegebene Bedeutung haben,

gegebenenfalls in Gegenwart eines Reaktionshilfsmittels und gegebenenfalls in Gegenwart eines Verdünnungsmittels umsetzt und gegebenenfalls die so erhaltenen Produkte der Formel (I) anschließend nach üblichen Methoden in andere Derivate gemäß der Definition der Verbindungen der Formel (I) umwandelt.

Die neuen Bisazinylverbindungen der Formel (I) zeichnen sich durch starke und selektive Herbizidwirkung aus.

Bevorzugte Substituenten bzw. Bereiche der in den oben und nachstehend erwähnten Formeln aufgeführten Reste werden im folgenden erläutert:

Alkyl steht in den allgemeinen Formeln einzeln oder in zusammengesetzten Resten wie z.B. Alkylamino, Alkoxycarbonylalkyl, für Alkyl mit vorzugsweise 1 bis 6, besonders bevorzugt 1 bis 4 und insbesondere 1 bis 2 Kohlenstoffatomen. Beispielhaft und vorzugsweise seien genannt Methyl, Ethyl, n- und iso-Propyl, n-, i-, s- und tert.-Butyl.

Alkoxy und Alkylthio stehen in den allgemeinen Formeln einzeln oder in zusammengesetzten Resten wie z.B. Alkoxycarbonyl, Alkylthiocarbonylalkyl, für Alkoxy bzw. Alkylthio mit vorzugsweise 1 bis 6, besonders bevorzugt 1 bis 4 und insbesondere bevorzugt 1 bis 2 Kohlenstoffatomen. Beispielhaft und vorzugsweise seien Methoxy, Ethoxy, n- und i-Propoxy, n-, i-, s- und tert.-Butoxy, Methylthio, Ethylthio, n-

und i-Propylthio, n-, i-, s-und tert.-Butylthio genannt.

Alkenyl und Alkinyl stehen in den allgemeinen Formeln einzeln oder in zusammengesetzten Resten für Alkenyl bzw. Alkinyl mit vorzugsweise 3 bis 6, besonders bevorzugt mit 3 oder 4 Kohlenstoffatomen. Beispielhaft und vorzugsweise seien genannt Allyl und Propargyl.

Halogenalkyl, Halogenalkoxy und Halogenalkylthio stehen in den allgemeinen Formeln für geradkettiges oder verzweigtes Halogenalkyl, Halogenalkoxy bzw. Halogenalkylthio mit 1 bis 4 und bevorzugt mit 1 oder 2 Kohlenstoffatomen und jeweils 1 bis 9 und bevorzugt 1 bis 5, gleichen oder verschiedenen Halogenatomen wie unter Halogen definiert; beispielhaft und vorzugsweise seien genannt:
Fluormethyl, Chlormethyl, Brommethyl, Fluorethyl, Chlorethyl, Bromethyl, Fluor-n-propyl, Chlor-n-propyl, Dichlormethyl, Difluorethyl, Trifluorethyl, Trichlorethyl, Trifluorchlorethyl, Chlorbutyl, Fluorbutyl und insbesondere Difluormethyl, Trifluormethyl, Trichlormethyl, Dichlorfluor-methyl und Chlordifluor-methyl sowie die diesen entsprechenden Halogenalkoxyreste bzw. Halogenalkylthioreste.

Die hier aufgeführten Definitionen gelten in entsprechender Weise auch für die im folgenden aufgeführten bevorzugten Kombinationen von Resten.

Die Erfindung betrifft vorzugsweise Verbindungen der Formel (I), in welcher

$A^1$ und $A^2$ gleich oder verschieden sind und für Stickstoff oder eine C-X-Gruppierung stehen, wobei X für Wasserstoff, Halogen, $C_1$-$C_4$-Alkyl oder $C_1$-$C_4$-Alkoxy steht,

$Q^1$ und $Q^2$ gleich oder verschieden sind und für Sauerstoff, Schwefel, NH oder N-$C_1$-$C_4$-Alkyl stehen,

R für Wasserstoff, Amino, Hydroxy, Cyano, Nitro, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino, Di-($C_1$-$C_4$-alkyl)-amino, $C_1$-$C_4$-Alkyl-carbonylamino, $C_1$-$C_4$-Alkoxy-carbonylamino oder $C_1$-$C_4$-Alkylsulfonylamino steht,

$X^1$, $X^2$, $Y^1$ und $Y^2$ gleich oder verschieden sind und für Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkylamino, Di-($C_1$-$C_4$-alkyl)-amino oder für Phenoxy stehen, welches gegebenenfalls durch Cyano, Nitro, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio und/oder $C_1$-$C_4$-Alkoxycarbonyl substituiert ist, und

Z für eine der nachstehenden Gruppierungen steht:

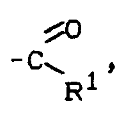,
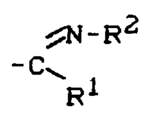,
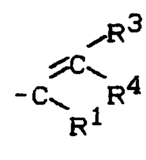,

wobei

$R^1$ für Wasserstoff, für gegebenenfalls durch Halogen oder $C_1$-$C_2$-Alkoxy substituiertes $C_1$-$C_4$-Alkyl oder für gegebenenfalls durch Halogen, $C_1$-$C_4$-Alkyl und/oder $C_1$-$C_4$-Alkoxy substituiertes Phenyl steht,

$R^2$ für Wasserstoff, Hydroxy, Amino oder für jeweils gegebenenfalls durch Halogen substituiertes $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxycarbonyl-$C_1$-$C_2$-alkoxy, Di-($C_1$-$C_4$-alkyl)-aminocarbonyl-$C_1$-$C_2$-alkoxy, $C_1$-$C_6$-Alkylamino, Di-($C_1$-$C_2$-alkyl)-amino, $C_1$-$C_6$-Alkylcarbonylamino, $C_1$-$C_6$-Alkoxycarbonylamino, $C_1$-$C_6$-Alkylsulfonylamino, oder für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxycarbonyl und/oder Di-($C_1$-$C_2$-alkyl)-amino substituiertes Phenyl, Phenyl-$C_1$-$C_4$-alkyl, Phenoxy, Phenyl-$C_1$-$C_4$-alkoxy, Phenylamino, Diphenylamino, Phenyl-$C_1$-$C_4$-alkylamino, N-($C_1$-$C_4$-alkyl)-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^3$ für Wasserstoff, Halogen, Cyano, Carboxy, $C_1$-$C_6$-Alkoxycarbonyl, $C_1$-$C_6$-Alkylcarbonylamino oder Di-($C_1$-$C_4$-alkoxy)-phosphoryl steht und

$R^4$ für Formyl, Cyano, Carboxy, Hydroxymethyl, Carbamoyl oder für jeweils gegebenenfalls durch Halogen, Carboxy oder $C_1$-$C_4$-Alkoxycarbonyl substituiertes $C_1$-$C_6$-

Alkoxycarbonyl, $C_5$-$C_6$-Cycloalkyloxycarbonyl, $C_1$-$C_6$-Alkylthiocarbonyl, $C_1$-$C_6$-Alkylaminocarbonyl oder $C_5$-$C_6$-Cycloalkylaminocarbonyl, für Di-($C_1$-$C_2$-alkyl)-aminocarbonyl, für $C_1$-$C_4$-Alkylamino-carbonyl-$C_1$-$C_4$-alkoxycarbonyl, für Di-($C_1$-$C_2$-alkyl)-aminocarbonyl-$C_1$-$C_4$-alkoxycarbonyl, für Phenylaminocarbonyl-$C_1$-$C_4$-alkoxycarbonyl, für N-Methyl-N-phenylaminocarbonyl-$C_1$-$C_4$-alkoxycarbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxycarbonyl und/oder Di-($C_1$-$C_2$-alkyl)-amino substituiertes Phenoxycarbonyl, Phenyl$C_1$-$C_4$-alkoxycarbonyl, Furylmethoxycarbonyl, Thienylmethoxycarbonyl, Phenylthiocarbonyl, Phenyl-$C_1$-$C_4$-alkylthiocarbonyl, Phenylaminocarbonyl, Phenyl-$C_1$-$C_4$-alkylamino-carbonyl, N-($C_1$-$C_4$-Alkyl)-N-phenylamino-carbonyl oder Phenylhydrazinocarbonyl, $C_1$-$C_4$-Alkylhydrazinocarbonyl, für Phthalimidoxycarbonyl oder zusammen mit $R^3$ für die Gruppierung $-CO-O-(CH_2)_n-$ steht, wobei

n für die Zahlen 1 bis 4, insbesondere 2 oder 3 steht.

Die in der Definition der erfindungsgemäßen Verbindungen aufgeführten aliphatischen Kohlenwasserstoffreste (z.B. Alkyl, Alkenyl, Alkinyl), auch in Kombination mit Heteroatomen (z.B. in Alkoxy, Alkylthio, Alkylamino) oder in Zusammensetzungen wie z.B. Halogenalkyl, Halogenalkoxy, sind jeweils geradkettig oder verzweigt.

Halogen steht im allgemeinen für Fluor, Chlor, Brom oder Jod, vorzugsweise für Fluor, Chlor oder Brom, insbesondere für Fluor oder Chlor.

Die Erfindung betrifft insbesondere Verbindungen der Formel (I), in welcher

$A^1$ und $A^2$ gleich oder verschieden sind und für Stickstoff oder eine C-X-Gruppierung stehen, wobei X für Wasserstoff, Fluor, Chlor, Brom, Methyl oder Methoxy steht,

$Q^1$ und $Q^2$ gleich oder verschieden sind und für Sauerstoff, Schwefel, NH oder $N-CH_3$ stehen,

R für Wasserstoff, Amino, Hydroxy, Fluor, Chlor, Brom, Methyl, Ethyl, Trifluormethyl, Methoxy, Difluormethoxy, Trifluormethoxy, Methylthio, Trifluormethylthio, Methylamino, Dimethylamino, Acetylamino, Methoxycarbonylamino oder Methylsulfonylamino steht,

$X^1$, $X^2$, $Y^1$ und $Y^2$ gleich oder verschieden sind und für Wasserstoff, Chlor, Methyl, Ethyl, Trifluormethyl, Methoxy, Ethoxy, Difluormethoxy, Methylthio, Methylamino, Ethylamino oder Dimethylamino stehen, und

Z für eine der nachsstehenden Gruppierungen steht

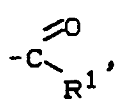 , 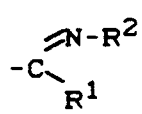 , 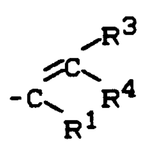

wobei

$R^1$ für Wasserstoff, Methyl, Ethyl, Trifluormethyl oder Methoxymethyl steht,

$R^2$ für Wasserstoff, Hydroxy, Amino, Methyl, Ethyl, n-Propyl, Isopropyl, n-Butyl, Isobutyl, sec-Butyl, tert-Butyl, Allyl, Propargyl, Methoxy, Ethoxy, n-Propoxy, Isopropoxy, n-Butoxy, Isobutoxy, sec-Butoxy, Allyloxy, Methoxycarbonylmethoxy, Ethoxycarbonylmethoxy, Methoxycarbonylethoxy, Ethoxycarbonylethoxy, Methylamino, Ethylamino, n-Propylamino, Isopropylamino, n-Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenyl, Benzyl, Phenoxy, Benzyloxy, Phenylamino, Benzylamino, N-Methyl-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^3$ für Wasserstoff, Fluor, Chlor, Cyano, Carboxy, $C_1$-$C_4$-Alkoxycarbonyl, $C_1$-$C_4$-Alkylcarbonylamino, Dimethoxyphosphoryl oder Diethoxyphosphoryl steht und

$R^4$ für Formyl, Cyano, Carboxy, Hydroxymethyl, Carbamoyl, für jeweils gegebenenfalls

durch Fluor, Chlor, Carboxy oder $C_1$-$C_4$-Alkoxycarbonyl substituiertes $C_1$-$C_4$-Alkoxycarbonyl, $C_5$-$C_6$-Cycloalkyloxycarbonyl, $C_1$-$C_4$-Alkylthiocarbonyl, $C_1$-$C_4$-Alkylaminocarbonyl oder $C_5$-$C_6$-Cycloalkylaminocarbonyl, für Dimethylaminocarbonyl, für $C_1$-$C_4$-Alkylaminocarbonyl-$C_1$-$C_4$-alkoxycarbonyl, für Dimethylaminocarbonyl-$C_1$-$C_4$-alkoxycarbonyl, für N-Methyl-N-phenylaminocarbonyl-$C_1$-$C_4$-alkoxycarbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-N-phenylaminocarbonyl, Phenylhydrazinocarbonyl, für Phthalimidoxycarbonyl oder zusammen mit $R^6$ für die Gruppierung $-CO-O-CH_2CH_2-$ steht.

Ganz besonders bevorzugt sind die Verbindungen der Formel (I), worin

$A^1$ und $A^2$ gleich oder verschieden sind und für Stickstoff oder eine CH-Gruppierung stehen,

$Q^1$ und $Q^2$ gleich oder verschieden sind und für Sauerstoff oder Schwefel stehen,

R für Wasserstoff, Fluor oder Chlor steht,

$X^1$, $X^2$, $Y^1$ und $Y^2$ gleich oder verschieden sind und für Chlor, Methyl, Methoxy oder Ethoxy stehen, und

Z die oben als insbesondere bevorzugt angegebene Bedeutung hat.

Verwendet man für das erfindungsgemäße Verfahren (a) zur Herstellung der Verbindungen der Formel (I) beispielsweise 2,6-Bis-(4,6-dimethoxy-s-triazin-2-yl-oxy)-benzyl-alkohol und Mangan(IV)-oxid als Ausgangsstoffe, so kann der Reaktionsablauf durch das folgende Formelschema wiedergegeben werden:

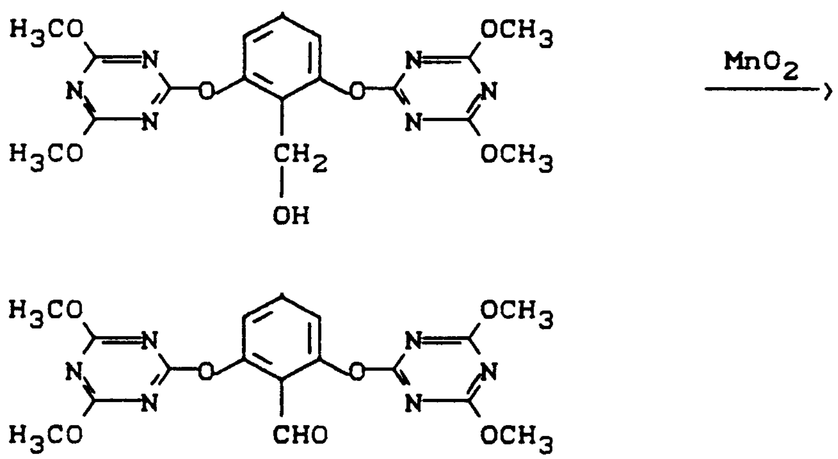


Verwendet man für das erfindungsgemäße Verfahren (b) beispielsweise 2-Mercapto-6-(4-methoxy-6-methyl-pyrimidin-2-yl-oxy)-benzaldehyd und 2-Chlor-4-methoxy-6-methyl-s-triazin als Ausgangsstoffe, so kann der Reaktionsablauf durch das folgende Formelschema wiedergegeben werden:

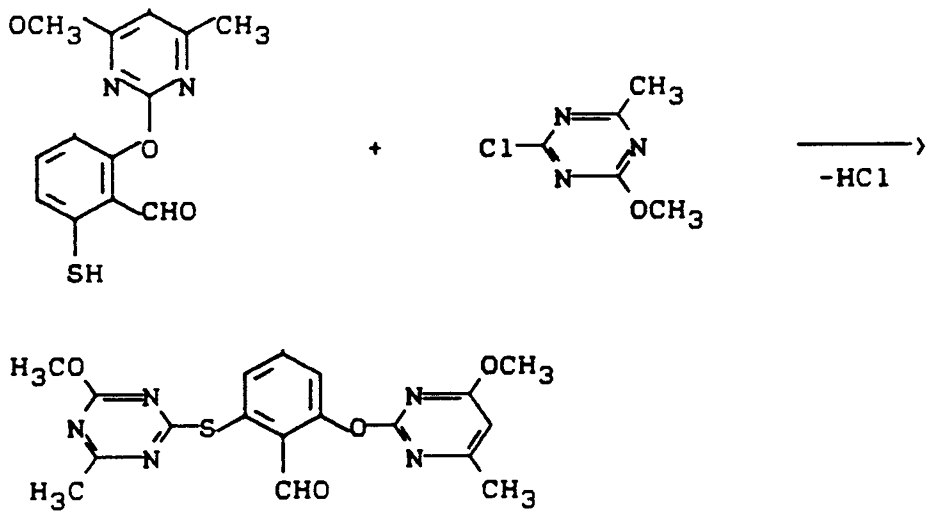

Verwendet man für das erfindungsgemäße Verfahren (c) beispielsweise 2,6-Bis-(4,6-dimethoxy-pyrimidin-2-yl-oxy)-acetophenonund Acethydrazid als Ausgangsstoffe, so kann der Reaktionsablauf durch das folgende Formelschema wiedergegeben werden:

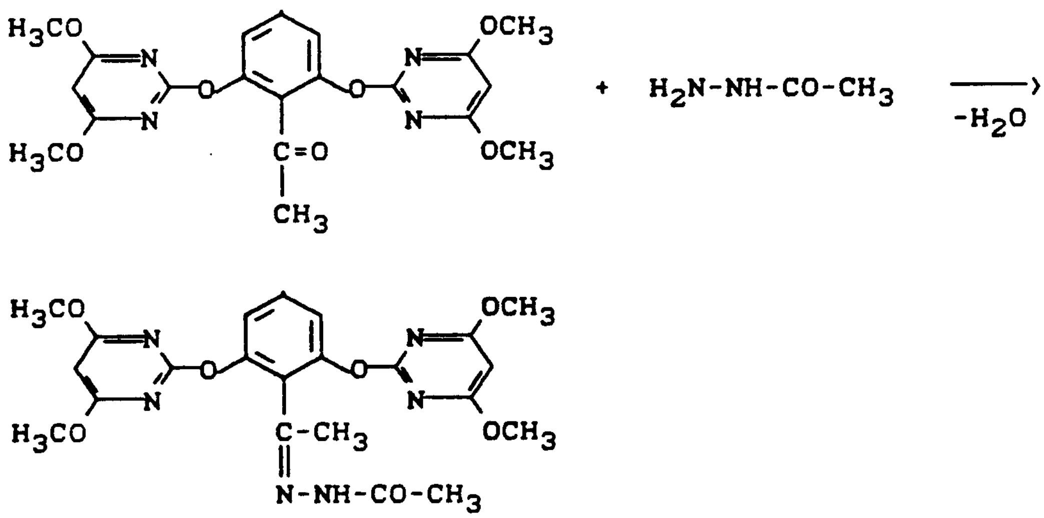

Die beim erfindungsgemäßen Verfahren (a) zur Herstellung von Verbindungen der Formel (I) als Ausgangsstoffe zu verwendenden Hydroxyverbindungen sind durch die Formel (II) allgemein definiert.

In Formel (II) haben $A^1$, $A^2$, $Q^1$, $Q^2$, R, $R^1$, $X^1$, $X^2$, $Y^1$ und $Y^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $A^1$, $A^2$, $Q^1$, $Q^2$, R, $R^1$, $X^1$, $X^2$, $Y^1$ und $Y^2$ angegeben wurden.

Als Beispiele für die Ausgangsstoffe der Formel (II) seien genannt:

2,6-Bis-(4,6-dimethyl-pyrimidin-2-yl-oxy)-, 2,6-Bis-(4-methoxy-6-methyl-pyrimidin-2-yl-oxy)-, 2,6-Bis-(4,6-dimethoxy-pyrimidin-2-yl-oxy)-, 2,6-Bis-(4,6-dimethyl-s-triazin-2-yl-oxy)-, 2,6-Bis-(4-methoxy-6-methyl-s-triazin-2-yl-oxy)- und 2,6-Bis-(4,6-dimethoxy-s-triazin-2-yl-oxy)-benzylalkohol und -$\alpha$-methyl-benzylalkohol.

Die Hydroxyverbindungen der Formel (II) sind noch nicht aus der Literatur bekannt und sind ebenfalls Gegenstand der vorliegenden Anmeldung.

Man erhält die neuen Verbindungen der Formel (II), wenn man entsprechende Alkylverbindungen der allgemeinen Formel (VII),

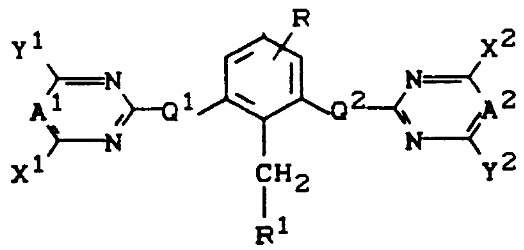

(VII)

in welcher

$A^1$, $A^2$, $Q^1$, $Q^2$, R, $R^1$, $X^1$, $X^2$, $Y^1$ und $Y^2$ die oben angegebene Bedeutung haben,

mit geeigneten Halogenierungsmitteln, wie z.B. N-Chlor- oder N-Brom-succinimid, gegebenenfalls in Gegenwart eines Katalysators, wie z.B. Dibenzoylperoxid und gegebenenfalls in Gegenwart eines Verdünnungsmittels, wie z.B. Tetrachlormethan, bei Temperaturen zwischen 0 °C und 150 °C umsetzt und die so erhaltenen Halogenalkylverbindungen der allgemeinen Formel (VIII),

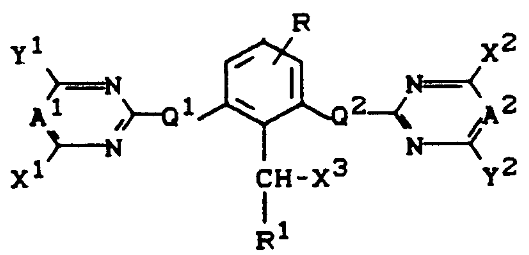

(VIII)

in welcher

$A^1$, $A^2$, $Q^1$, $Q^2$, R, $R^1$, $X^1$, $X^2$, $Y^1$ und $Y^2$ die oben angegebene Bedeutung haben und

$X^3$ für Chlor oder Brom steht,

mit einem geeigneten Hydroxylierungsmittel, wie z.B. Silbernitrat/Wasser, gegebenenfalls in Gegenwart eines organischen Lösungsmittels, wie z.B. Aceton, bei Temperaturen zwischen 0 °C und 100 °C umsetzt (vgl. die Herstellungsbeispiele).

In den Formeln (VII) und (VIII) haben $A^1$, $A^2$, $Q^1$, $Q^2$, R, $R^1$, $X^1$, $X^2$, $Y^1$ und $Y^2$ jeweils vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $A^1$, $A^2$, $Q^1$, $Q^2$, R, $R^1$, $X^1$, $X^2$, $Y^1$ und $Y^2$ angegeben wurden.

Die Alkylverbindungen der Formel (VII) sind bekannt und/oder können nach an sich bekannten Verfahren hergestellt werden (vgl. EP-A 008192 und die Herstellungsbeispiele).

Die Halogenalkylverbindungen der Formel (VIII), deren Herstellung oben beschrieben wurde (vgl. auch die Herstellungsbeispiele) sind noch nicht aus der Literatur bekannt und sind Gegenstand der vorliegenden Erfindung.

Das erfindungsgemäße Verfahren (a) wird unter Verwendung von Oxidationsmitteln durchgeführt. Als Oxidationsmittel werden hierbei vorzugsweise die üblicherweise für die Dehydrierung von Alkoholen zu Aldehyden oder Ketonen verwendeten Stoffe, wie z.B. Mangan(IV)-oxid ("Braunstein"), Dimethylsulfoxid/Oxalylchlorid (Swern-Reagenz), Chrom(VI)-oxid oder Natriumdichromat/Schwefelsäure, eingesetzt.

Das erfindungsgemäße Verfahren (a) zur Herstellung der neuen Bisazinylverbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im allgemeinen arbeitet man bei Temperaturen zwischen -80 °C und +150 °C, vorzugsweise bei Temperaturen zwischen -60 °C und +100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man auf 1 Mol Hydroxyverbindung der Formel (II) im allgemeinen zwischen 1 und 50, vorzugsweise zwischen 1 und 25 Moläquivalenten, des Oxidationsmittels ein.

Im allgemeinen wird die Ausgangsverbindung der Formel (II) in einem geeigneten Verdünnungsmittel vorgelegt und das Oxidationsmittel langsam dazu gegeben. Das Reaktionsgemisch wird bei der erforderlichen Temperatur bis zum Ende der Umsetzung gerührt und auf übliche Weise aufgearbeitet (vgl. Herstellungsbeispiele).

Die beim erfindungsgemäßen Verfahren (b) zur Herstellung von Verbindungen der Formel (I) als Ausgangsstoffe zu verwendenden Benzolderivate sind durch die Formel (III) allgemein definiert.

In Formel (III) haben $Q^1$, $Q^2$, R und $R^1$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $Q^1$, $Q^2$, R und $R^1$ angegeben wurden;

$E^1$ steht vorzugsweise für Wasserstoff oder die Gruppierung

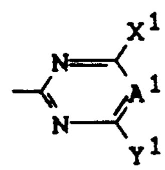

wobei $A^1$, $X^1$ und $Y^1$ vorzugsweise bzw. insbesondere diejenigen Bedeutungen haben, die bereits oben im Zusammenhang mit der Beschreibung der erfindungsgemäßen Verbindungen der Formel (I) vorzugsweise bzw. als insbesondere bevorzugt für $A^1$, $X^1$ und $Y^1$ angegeben wurden.

Als Beispiele für die Ausgangsstoffe der Formel (III) seien genannt:
2-(4,6-Dimethyl-pyrimidin-2-yl-oxy)-, 2-(4-Methoxy-6-methyl-pyrimidin-2-yl-oxy)-, 2-(4,6-Dimethoxy-pyrimidin-2-yl-oxy)-, 2-(4,6-Dimethyl-s-triazin-2-yl-oxy)-, 2-(4-Methoxy-6-methyl-s-triazin-2-yl-oxy)- und 2-(4,6-Dimethoxy-s-triazin-2-yl-oxy)-6-hydroxy-benzaldehyd und -6-hydroxy-acetophenon sowie 2,6-Dihydroxy-benzaldehyd und 2,6-Dihydroxy-acetophenon.

Die Benzolderivate der Formel (III) sind teilweise bekannt (vgl. JP-A 6910256/JP-A 4410256 - zitiert in Chem. Abstracts 71, 112633f; J, Med. Chem. 20 (1977), 1194-1199).

Neu und Gegenstand der vorliegenden Anmeldung sind die Verbindungen der Formel (IIIa),

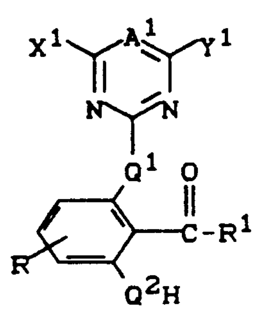

(IIIa)

in welcher

$A^1$, $Q^1$, $Q^2$, R, $R^1$, $X^1$ und $Y^1$ die oben angegebene Bedeutung haben.

Man erhält die neuen Verbindungen der allgemeinen Formel (IIIa), wenn man Carbonsäurederivate der allgemeinen Formel (IX),

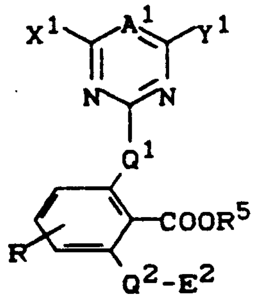

(IX)

in welcher

$A^1$, $Q^1$, $Q^2$, R, $X^1$ und $Y^1$ die oben angegebene Bedeutung haben,

$E^2$ für Wasserstoff oder die Gruppierung

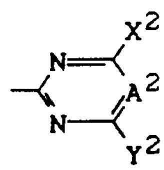

steht, wobei

$A^2$, $X^2$ und $Y^2$ die oben angegebene Bedeutung haben, und

$R^5$ für Wasserstoff oder Niederalkyl steht,

mit Reduktionsmitteln, wie z.B. Natriumborhydrid oder Natrium-bis-(2-methoxyethoxy)-dihydroaluminat ("Red-Al®"), gegebenenfalls in Gegenwart von Verdünnungsmitteln, wie z.B. Diethylether, Dimethoxyethan, Tetrahydrofuran, Toluol, ggf. auch Methanol, Ethanol oder Isopropanol, und gegebenenfalls in Gegenwart eines Reaktionshilfsmittels, wie z.B. N-Methyl-piperazin, bei Temperaturen zwischen -70 °C und +50 °C umsetzt und auf übliche Weise aufarbeitet (vgl. die Herstellungsbeispiele).

In Formel (IX) haben $A^1$, $Q^1$, $Q^2$, R, $X^1$ und $Y^1$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $A^1$, $Q^1$, $Q^2$, R, $X^1$ und $Y^1$ angegeben wurden; $E^2$ steht vorzugsweise für Wasserstoff oder die Gruppierung

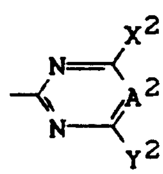

wobei $A^2$, $X^2$ und $Y^2$ vorzugsweise bzw. insbesondere diejenigen Bedeutungen haben, die bereits oben im Zusammenhang mit der Beschreibung der erfindungsgemäßen Verbindungen der Formel (I) vorzugsweise bzw. als insbesondere bevorzugt für $A^2$, $X^2$ und $Y^2$ angegeben wurden,

$R^5$ steht vorzugsweise für Wasserstoff, Methyl oder Ethyl.

Die Carbonsäurederivate der Formel (IX) sind bekannt und/oder können nach an sich bekannten Verfahren hergestellt werden (vgl. EP-A 249708; EP-A 321846; EP-A 336494).

Die beim erfindungsgemäßen Verfahren (b) zur Herstellung von Verbindungen der Formel (I) weiter als Ausgangsstoffe zu verwendenden reaktionsfähigen Azine sind durch die Formeln (IVa) und (IVb) allgemein definiert.

In den Formeln (IVa) und (IVb) haben $A^1$, $A^2$, $X^1$, $X^2$, $Y^1$ und $Y^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $A^1$, $A^2$, $X^1$, $X^2$, $Y^1$ und $Y^2$ angegeben wurden und

G steht vorzugsweise für Fluor, Chlor, Brom oder $C_1$-$C_4$-Alkylsulfonyl, insbesondere für Chlor oder Methylsulfonyl.

Als Beispiele für die Ausgangsstoffe der Formeln (IVa) und (IVb) seien genannt:
2-Chlor- und 2-Methylsulfonyl-4,6-dimethyl-pyrimidin, -4-methyl-6-methoxy-pyrimidin, -4,6-dimethoxy-pyrimidin, -4-methyl-6-ethoxy-pyrimidin, -4-chlor-6-methoxy-pyrimidin, -4-methyl-pyrimidin, -4-chlor-6-methyl-pyrimidin, -4-trifluormethyl-6-methoxy-pyrimidin, -4-methoxy-6-difluormethoxy-pyrimidin, -4-methyl-6-difluormethoxy-pyrimidin, -4,6-bis-difluormethoxy-pyrimidin, -4-chlor-6-ethoxy-pyrimidin, -4,6-diethoxy-pyrimidin, -4,5-dichlor-6-methyl-pyrimidin, -4-methyl-5-chlor-6-methoxy-pyrimidin, -4,6-dichlor-pyrimidin, -4-ethyl-6-methoxy-pyrimidin, -5-chlor-4,6-dimethoxy-pyrimidin, -4-methoxy-6-methylamino-pyrimidin und -4,6-bis-trifluormethyl-pyrimidin, sowie 2-Chlor-4,6-dimethyl-s-triazin, -4-methoxy-6-methyl-s-triazin, -4,6-dimethoxy-s-triazin, -4-ethoxy-6-methyl-s-triazin und -4-ethyl-6-methoxy-s-triazin.

Die reaktionsfähigen Azine der Formeln (IVa) und (IVb) sind bekannt und/oder können nach an sich bekannten Verfahren hergestellt werden (vgl. J. Chem. Soc. 1957, 1830, 1833; J. Org. Chem. 26 (1961), 792; US-P 3308119; US-P 4711959).

Das erfindungsgemäße Verfahren (b) wird vorzugsweise unter Verwendung eines Verdünnungsmittels durchgeführt. Es kommen hierbei die gleichen Verdünnungsmittel in Betracht, die oben für das erfindungsgemäße Verfahren (a) angegeben sind.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calciumhydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und -tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Die beim erfindungsgemäßen Verfahren (c) zur Herstellung von Verbindungen der Formel (I) als Ausgangsstoffe zu verwendenden Carbonylverbindungen sind durch die Formel (Ia) allgemein definiert.

In Formel (Ia ) haben $A^1$, $A^2$, $Q^1$, $Q^2$, R, $R^1$, $X^1$, $X^2$, $Y^1$ und $Y^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $A^1$, $A^2$, $Q^1$, $Q^2$, R, $R^1$, $X^1$, $X^2$, $Y^1$ und $Y^2$ angegeben wurden.

Als Beispiele für die Ausgangsstoffe der Formel (Ia) seien genannt:
2,6-Bis-(4,6-dimethyl-pyrimidin-2-yl-oxy)-, 2,6-Bis-(4,6-dimethoxy-pyrimidin-2-yl-oxy)-, 2,6-Bis-(4-methoxy-6-methyl-pyrimidin-2-yl-oxy)-, 2,6-Bis-(4,6-dimethyl-s-triazin-2-yl-oxy)-, 2,6-Bis-(4,6-dimethoxy-s-triazin-s-yl-oxy)- und 2,6-Bis-(4-methoxy-6-methyl-s-triazin-2-yl-oxy)-benzaldehyd und -acetophenon.

Die Carbonylverbindungen der Formel (Ia) sind erfindungsgemäße, neue Verbindungen; sie können nach den erfindungsgemäßen Verfahren (a) und (b) hergestellt werden.

Die beim erfindungsgemäßen Verfahren (c) weiter als Ausgangsstoffe zu verwendenden Aminoverbindungen oder Methylenverbindungen sind durch die Formeln (V) und (VI) allgemein definiert. In diesen Formeln haben $R^2$, $R^3$ und $R^4$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^2$, $R^3$ und $R^4$ angegeben wurde.

Als Beispiele für die Ausgangsstoffe der Formeln (V) und (VI) seien genannt:
Ammoniak, Hydroxylamin, Hydrazin, Methylamin, Ethylamin, Propylamin, Isopropylamin, Butylamin, Isobutylamin, sec-Butylamin, tert-Butylamin, Allylamin, Propargylamin, O-Methyl-, O-Ethyl-, O-Propyl-, O-Isopropyl-, O-Butyl-, O-Isobutyl- und O-sec-Butyl-hydroxylamin, O-Allyl-hydroxylamin, Aminooxyessigsäure-methylester und -ethylester, α-Aminooxy-propionsäure-methylester und -ethylester, Methylhydrazin, Ethylhydrazin, Propylhydrazin, Isopropylhydrazin, Butylhydrazin, Isobutylhydrazin, sec-Butyl-hydrazin, tert-Butylhydrazin, N,N-Dimethylhydrazin, Acethydrazid, Propionylhydrazid, Methoxycarbonylhydrazin, Ethoxycarbonylhydrazin, Methylsulfonylhydrazin, Ethylsulfonylhydrazin, Phenylhydrazin, Benzoylhydrazin, Benzolsulfonsäurehydrazid, p-Toluolsulfonsäurehydrazid, Malonsäure, Cyanessigsäure, Malonsäuredinitril, Cyanessigsäure-methylester und -ethylester, Malonsäure-dimethylester und -diethylester, γ-Butyrolacton.

Die Ausgangsstoffe der Formeln (V) und (VI) sind bekannte Synthesechemikalien.

Das erfindungsgemäße Verfahren (c) wird vorzugsweise unter Verwendung eines Verdünnungsmittels durchgeführt. Es kommen hierbei die gleichen Verdünnungsmittel in Betracht, die oben für das erfindungsgemäße Verfahren (a) angegeben sind.

Das erfindungsgemäße Verfahren (c) wird gegebenenfalls in Gegenwart eines Reaktionshilfsmittels durchgeführt. Geeignete Reaktionshilfsmittel sind Stoffe, die üblicherweise zur Steuerung und/oder Beschleunigung von Kondensationsreaktionen zwischen Carbonylverbindungen und Amino- oder Methylen-Verbindungen verwendet werden. Hierzu gehören insbesondere Stickstoffverbindungen, wie z.B. Ammoniumacetat, β-Alanin, Pyridin und Piperidin.

Die Reaktionstemperaturen können bei dem erfindungsgemäßen Verfahren (c) in einem größeren Bereich variiert werden. Im allgemeinen arbeitet man bei Temperaturen zwischen 0 °C und 150 °C, vorzugsweise bei Temperaturen zwischen 10 °C und 120 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden, Die Reaktionen werden im allgemeinen in einem geeigneten Verdünnungsmittel, gegebenenfalls in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vergleiche die Herstellungsbeispiele).

Die erfindungsgemäßen Wirkstoffe können als Defoliants, Desiccants, Krautabtötungsmittel und insbesondere als Unkrautvernichtungsmittel verwendet werden, Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen, wie z.B. Weizen, sowohl im Vorauflauf- als auch im

Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage:

z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methyl-thio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSUL-FURON); N,N-Dimethyl-N'-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxy]-phenoxy)-propansäure, deren Methyl-oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 4-Ethylamino-2-t-butylamino-6-methylthio-s-triazin(TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE).

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken, Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Nach den oben allgemein beschriebenen Herstellungsverfahren (a) und (b) erhält man Endprodukte der Formel (I), worin Z für

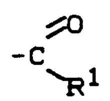

steht,
während nach Verfahren (c) Endprodukte der Formel (I) erhalten werden, worin Z für

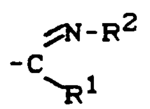

oder

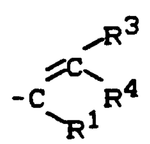

steht.

Herstellungsbeispiele:

Beispiel 1

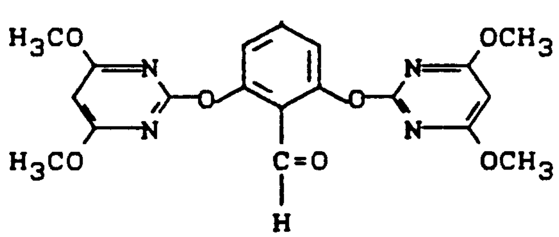

(Verfahren (b))

Eine Mischung aus 1,1 g (4 mmol) 2-(4,6-Dimethoxy-pyrimidin-2-yl-oxy)-6-hydroxy-benzaldehyd, 0,9 g (4,2 mmol) 4,6-Dimethoxy-2-methylsulfonyl-pyrimidin, 1,2 g (9 mmol) Kaliumcarbonat und 40 ml Acetonitril wird 5 Stunden unter Rückfluß erhitzt und anschließend eingeengt. Der Rückstand wird in Methyl-tert-butylether aufgenommen, zweimal mit Wasser gewaschen, mit Natriumsulfat getrocknet und filtriert. Das Filtrat wird eingeengt, der Rückstand mit Diethylether verrieben und das kristalline Produkt durch Absaugen isoliert.

Man erhält 0,9 g (54% der Theorie) 2,6-Bis-(4,6-dimethoxy-pyrimidin-2-yl-oxy)-benzaldehyd vom Schmelzpunkt 129 ° C.

Beispiel 2

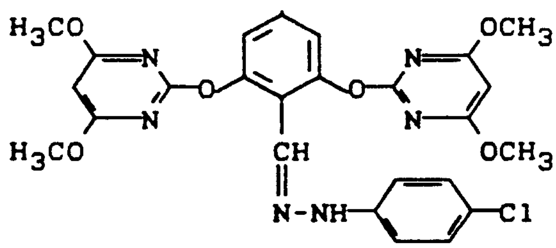

(Verfahren (c))

Eine Mischung aus 1,24 g (3 mmol) 2,6-Bis-(4,6-dimethoxy-pyrimidin-2-yl-oxy)-benzaldehyd, 0,57 g (3,2 mmol) 4-Chlor-phenylhydrazin-hydrochlorid, 0,3 g (3,7 mmol) Natriumacetat und 60 ml Methylenchlorid wird 15 Stunden bei 20 ° C gerührt, dann mit einer 5%igen Dinatriumhydrogenphosphat-Lösung gewaschen, mit Natriumsulfat getrocknet und filtriert. Das Filtrat wird eingeengt, der Rückstand mit Hexan verrührt und das kristalline Produkt durch Absaugen isoliert.

Man erhält 1,1 g (68% der Theorie) 2,6-Bis-(4,6-dimethoxy-pyrimidin-2-yl-oxy)-benzaldehyd-(4-chlorphenylhydrazon) vom Schmelzpunkt 140 ° C.

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

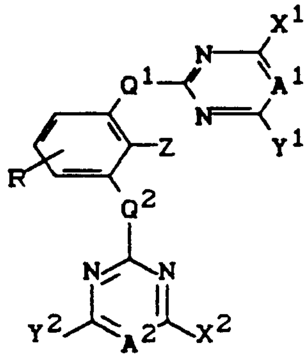

(I)

**Tabelle 1:** Beispiele für die Verbindungen der Formel (I)

| Bsp.-Nr. | $A^1$ | $A^2$ | $Q^1$ | $Q^2$ | R | $X^1$ | $X^2$ | $Y^1$ | $Y^2$ | Z | Schmelzpunkt (°C) |
|---|---|---|---|---|---|---|---|---|---|---|---|
| 3 | CH | CH | O | O | H | $OCH_3$ | $OCH_3$ | $OCH_3$ | $OCH_3$ | 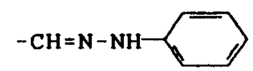 | 115 |
| 4 | CH | CH | O | O | H | $OCH_3$ | $OCH_3$ | $OCH_3$ | $OCH_3$ | 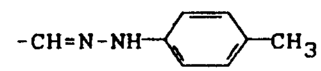 | 121 |
| 5 | CH | CH | O | O | H | $OCH_3$ | $OCH_3$ | $OCH_3$ | $OCH_3$ | 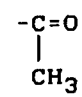 | 124 |
| 6 | CH | CH | O | O | H | $OCH_3$ | $OCH_3$ | $OCH_3$ | $OCH_3$ | 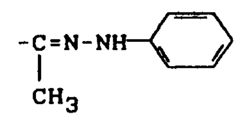 | 80 |

Tabelle 1: Beispiele für die Verbindungen der Formel (I) - Fortsetzung -

| Bsp.-Nr. | $A^1$ | $A^2$ | $Q^1$ | $Q^2$ | R | $X^1$ | $X^2$ | $Y^1$ | $Y^2$ | Z | Schmelzpunkt (°C) |
|---|---|---|---|---|---|---|---|---|---|---|---|
| 7 | CH | CH | O | O | H | $OCH_3$ | $OCH_3$ | $OCH_3$ | $OCH_3$ | -CH=N-NH- 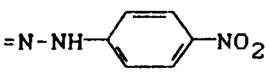 | 121 |
| 8 | CH | CH | O | O | H | $OCH_3$ | $OCH_3$ | $OCH_3$ | $OCH_3$ | -CH=N-NH- 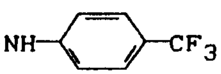 | 150 |
| 9 | CH | CH | O | O | H | $OCH_3$ | $OCH_3$ | $OCH_3$ | $OCH_3$ | -CH=N-NH- 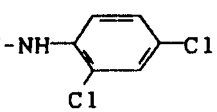 | 168 |
| 10 | CH | CH | O | O | H | $OCH_3$ | $OCH_3$ | $OCH_3$ | $OCH_3$ | -CH=N-NH- 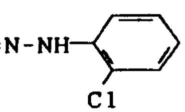 | 217 |
| 11 | CH | CH | O | O | H | $OCH_3$ | $OCH_3$ | $OCH_3$ | $OCH_3$ | -CH=N-NH- 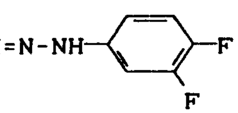 | 130 |

<u>Tabelle 1:</u> **Beispiele für die Verbindungen der Formel (I) - Fortsetzung -**

| Bsp.-Nr. | $A^1$ | $A^2$ | $Q^1$ | $Q^2$ | R | $X^1$ | $X^2$ | $Y^1$ | $Y^2$ | Z | Schmelzpunkt (°C) |
|---|---|---|---|---|---|---|---|---|---|---|---|
| 12 | CH | CH | O | O | H | $OCH_3$ | $OCH_3$ | $OCH_3$ | $OCH_3$ | -CH=N-NH- 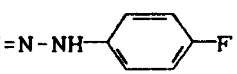 | 136 |
| 13 | CH | CH | O | O | H | $OCH_3$ | $OCH_3$ | $OCH_3$ | $OCH_3$ | -CH=N-NH- 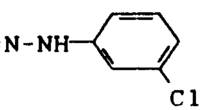 | 113 |
| 14 | CH | CH | O | O | H | $OCH_3$ | $OCH_3$ | $OCH_3$ | $OCH_3$ | -CH=N-NH- 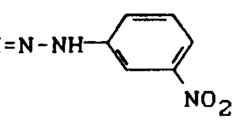 | 165 |
| 15 | CH | CH | O | O | H | $OCH_3$ | $OCH_3$ | $OCH_3$ | $OCH_3$ | -CH=N-NH- 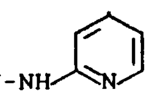 | 147 |
| 16 | CH | CH | O | O | H | $OCH_3$ | $OCH_3$ | $OCH_3$ | $OCH_3$ | -CH=N-NH- 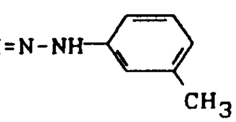 | 115 |

Tabelle 1: Beispiele für die Verbindungen der Formel (I) - Fortsetzung -

| Bsp.-Nr. | $A^1$ | $A^2$ | $Q^1$ | $Q^2$ | R | $X^1$ | $X^2$ | $Y^1$ | $Y^2$ | Z | Schmelzpunkt (°C) |
|---|---|---|---|---|---|---|---|---|---|---|---|
| 17 | CH | CH | O | O | H | $OCH_3$ | $OCH_3$ | $OCH_3$ | $OCH_3$ | -CH=N-NH- 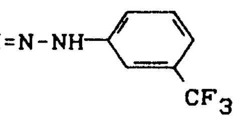 | 115 |
| 18 | CH | CH | O | O | H | $OCH_3$ | $OCH_3$ | $OCH_3$ | $OCH_3$ | -CH=N-NH- 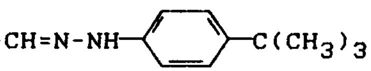 | 123 |
| 19 | CH | CH | O | O | H | $OCH_3$ | $OCH_3$ | $OCH_3$ | $OCH_3$ | -CH=N-NH- 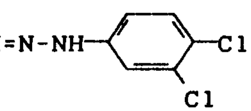 | 177 |
| 20 | CH | CH | O | O | H | $OCH_3$ | $OCH_3$ | $OCH_3$ | $OCH_3$ | $-CH=N-N(CH_3)_2$ | 85 |
| 21 | CH | CH | O | O | H | $OCH_3$ | $OCH_3$ | $OCH_3$ | $OCH_3$ | -CH=N-NH- 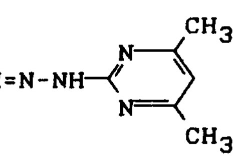 | 160 |

Tabelle 1: Beispiele für die Verbindungen der Formel (I) - Fortsetzung -

| Bsp.-Nr. | $A^1$ | $A^2$ | $Q^1$ | $Q^2$ | R | $X^1$ | $X^2$ | $Y^1$ | $Y^2$ | Z | Schmelzpunkt (°C) |
|---|---|---|---|---|---|---|---|---|---|---|---|
| 22 | CH | CH | O | O | H | $OCH_3$ | $OCH_3$ | $OCH_3$ | $OCH_3$ | $-CH=N-NH-SO_2-CH_3$ | 171 |
| 23 | CH | CH | O | O | H | $OCH_3$ | $OCH_3$ | $OCH_3$ | $OCH_3$ | $-CH=N-NH-SO_2-$ 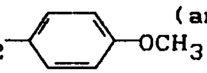 | (amorph) |
| 24 | CH | CH | O | O | H | $OCH_3$ | $OCH_3$ | $OCH_3$ | $OCH_3$ | $-CH=N-OCH_3$ | 126 |
| 25 | CH | CH | O | O | H | $OCH_3$ | $OCH_3$ | $OCH_3$ | $OCH_3$ | -CH=N-OH | 157 |
| 26 | CH | CH | O | O | H | $OCH_3$ | $OCH_3$ | $OCH_3$ | $OCH_3$ | $-CH=N-NH-SO_2-$ 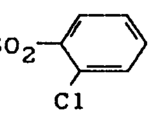 | 158 |
| 27 | CH | CH | O | O | H | $OCH_3$ | $OCH_3$ | $OCH_3$ | $OCH_3$ | $-CH=N-NH-SO_2-$ 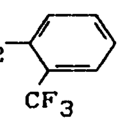 | 175 |

Tabelle 1: Beispiele für die Verbindungen der Formel (I) - Fortsetzung -

| Bsp.-Nr. | $A^1$ | $A^2$ | $Q^1$ | $Q^2$ | R | $X^1$ | $X^2$ | $Y^1$ | $Y^2$ | Z | Schmelzpunkt (°C) |
|---|---|---|---|---|---|---|---|---|---|---|---|
| 28 | CH | CH | O | O | H | $OCH_3$ | $OCH_3$ | $OCH_3$ | $OCH_3$ | $-CH=N-O-CH_2-CO-N(C_3H_7)_2$ | (amorph) |
| 29 | CH | CH | O | O | H | $OCH_3$ | $OCH_3$ | $OCH_3$ | $OCH_3$ | $-CH=N-N(C_6H_5)_2$ | 142 |
| 30 | CH | CH | O | O | H | $OCH_3$ | $OCH_3$ | $OCH_3$ | $OCH_3$ | -CH=N-NH- 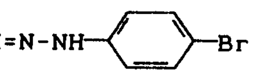 | 148 |
| 31 | CH | CH | O | O | H | $OCH_3$ | $OCH_3$ | $OCH_3$ | $OCH_3$ | -CH=N-NH- 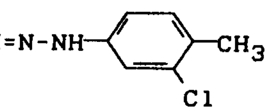 | 134 |
| 32 | CH | CH | O | O | H | $OCH_3$ | $OCH_3$ | $OCH_3$ | $OCH_3$ | -CH=N-NH- 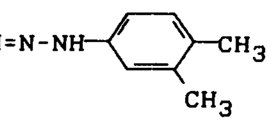 | 120 |
| 33 | CH | CH | O | O | H | $OCH_3$ | $OCH_3$ | $OCH_3$ | $OCH_3$ | -CH=N-NH- 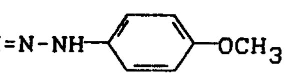 | 127 |

Tabelle 1: Beispiele für die Verbindungen der Formel (I) - Fortsetzung -

| Bsp.-Nr. | $A^1$ | $A^2$ | $Q^1$ | $Q^2$ | R | $X^1$ | $X^2$ | $Y^1$ | $Y^2$ | Z | Schmelzpunkt (°C) |
|---|---|---|---|---|---|---|---|---|---|---|---|
| 34 | CH | CH | O | O | H | $OCH_3$ | $OCH_3$ | $OCH_3$ | $OCH_3$ | 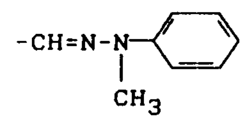 | 152 |
| 35 | CH | CH | O | O | H | $OCH_3$ | $OCH_3$ | $OCH_3$ | $OCH_3$ | $-CH=N-NH-CO-CH_3$ | 140 |
| 36 | CH | CH | O | O | H | $OCH_3$ | $OCH_3$ | $OCH_3$ | $OCH_3$ | 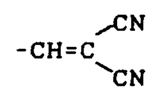 | 144 |
| 37 | CH | CH | O | O | H | $OCH_3$ | $OCH_3$ | $OCH_3$ | $OCH_3$ | 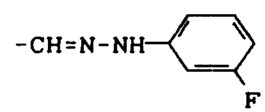 | 114 |

Ausgangsstoffe der Formel (II):

Beispiel (II-1)

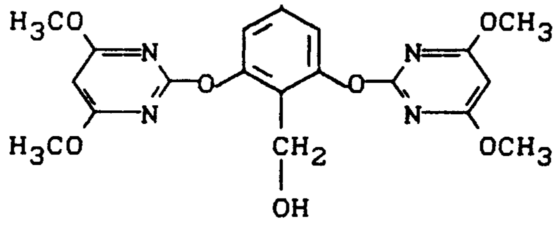

Stufe 1:

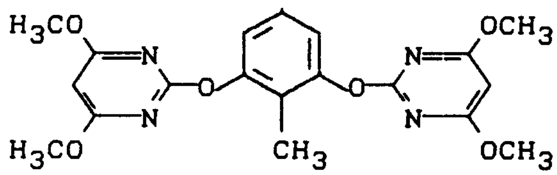

Eine Mischung aus 49,7 g (0,4 Mol) 2-Methyl-resorcin, 178,8 g (0,82 Mol) 4,6-Dimethoxy-2-methylsulfonyl-pyrimidin, 124,4 g (0,9 Mol) Kaliumcarbonat und 1 Liter Acetonitril wird 2 Tage unter Rückfluß erhitzt und dann eingeengt. Der Rückstand wird mit Wasser verrührt und das kristalline Produkt durch Absaugen (Nachwaschen mit Methanol) isoliert.

Man erhält 135 g (84% der Theorie) 2,6-Bis-(4,6-dimethoxy-pyrimidin-2-yl-oxy)-toluol vom Schmelzpunkt 140 ° C.

Stufe 2:

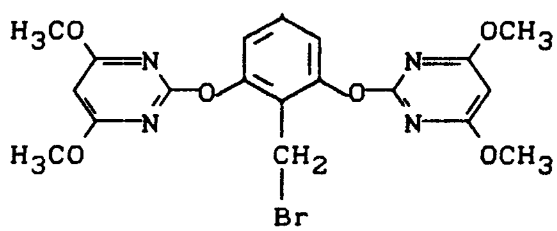

Eine Mischung aus 10,0 g (0,025 Mol) 2,6-Bis-(4,6-dimethoxy-pyrimidin-2-yl-oxy)-toluol, 4,45 g (0,025 Mol) N-Brom-succinimid, 140 ml Tetrachlormethan und einer Spatelspitze Dibenzoylperoxid wird 15 Stunden unter Rückfluß erhitzt, dann (nach Abkühlen) filtriert, zweimal mit Wasser gewaschen, mit Natriumsulfat getrocknet und filtriert. Das Filtrat wird eingeengt, der Rückstand mit Petrolether verrührt und das kristalline Produkt durch Absaugen isoliert.

Man erhält 9,0 g (75% der Theorie) 2,6-Bis-(4,6-dimethoxy-pyrimidin-2-yl-oxy)-benzylbromid vom Schmelzpunkt 127 ° C.

Stufe 3:

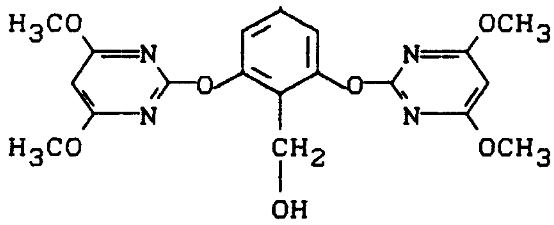

Eine Mischung aus 4,8 g (0,01 Mol) 2,6-Bis-(4,6-dimethoxy-pyrimidin-2-yl-oxy)-benzylbromid, 4,55 g (0,027 Mol) Silbernitrat, 40 ml Wasser und 40 ml Aceton wird 2 Stunden bei 20 ° C gerührt und dann filtriert. Vom Filtrat wird nach Verdünnen mit Essigsäureethylester die organische Phase abgetrennt, die wäßrige Phase noch zweimal mit Essigsäureethylester nachextrahiert; die organischen Phasen werden vereinigt, mit Natriumsulfat getrocknet und filtriert. Das Filtrat wird eingeengt und der Rückstand wird durch Säulenchromatographie (Kieselgel; Toluol/Aceton, 9:1) gereinigt.

Man erhält 1,4 g (34% der Theorie) 2,6-Bis-(4,6-dimethoxy-pyrimidin-2-yl-oxy)-benzylalkohol vom Schmelzpunkt 122 ° C.

Ausgangsstoffe der Formel (III):

Beispiel (III-1)

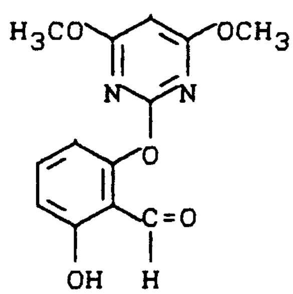

51,6 ml Red-Al® (3,4-molare Lösung von Natrium-bis-(2-methoxyethoxy)aluminiumdihydrid - 175 mmol - in Toluol) werden auf 0 ° C abgekühlt und mit einer Lösung von 19,3 g (193 mmol) N-Methyl-piperazin in 70 ml Toluol versetzt. Nach Abkühlen auf -60 ° C werden 23,4 g (53 mmol) 2,6-Bis-(4,6-dimethoxy-pyrimidin-2-yl-oxy)-benzoesäuremethylester in 280 ml Toluol dazugegeben und die Mischung wird 5 Stunden bei -60 ° C gerührt. Man läßt über Nacht auf Raumtemperatur kommen, gibt Wasser dazu und filtriert über Kieselgur. Vom Filtrat wird die organische Phase abgetrennt, die wäßrige Phase zweimal mit Essigsäureethylester nachextrahiert; die organischen Phasen werden vereinigt, mit Natriumsulfat getrocknet und filtriert. Vom Filtrat wird das Lösungsmittel im Wasserstrahlvakuum sorgfältig abdestilliert.

Man erhält 8 g (55% der Theorie) 2-(4,6-Dimethoxy-pyrimidin-2-yl-oxy)-6-hydroxy-benzaldehyd vom Schmelzpunkt 82 ° C.

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen (1) und (2) bei guter Verträglichkeit gegenüber Kulturpflanzen, wie z.B. Weizen, starke Wirkung gegen Unkräuter.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen beispielsweise die Verbindungen gemäß den Herstellungsbeispielen (1) und (2) bei guter Verträglichkeit gegenüber Kulturpflanzen, wie z.B. Weizen, starke Wirkung gegen Unkräuter.

## Patentansprüche

**1.** Bisazinylverbindungen der allgemeinen Formel (I),

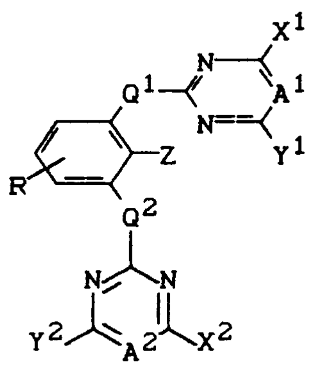

(I)

in welcher

| | |
|---|---|
| $A^1$ und $A^2$ | gleich oder verschieden sind und für Stickstoff oder eine C-X-Gruppierung stehen, wobei X für Wasserstoff, Halogen, Alkyl oder Alkoxy steht, |
| $Q^1$ und $Q^2$ | gleich oder verschieden sind und für Sauerstoff, Schwefel, NH oder N-Alkyl stehen, |
| R | für Wasserstoff, Amino, Hydroxy, Cyano, Nitro, Halogen, Alkyl, Halogenalkyl, Alkoxy, Halogenalkoxy, Alkylthio, Halogenalkylthio, Alkylamino, Dialkylamino, Alkylcarbonylamino, Alkoxycarbonylamino oder Alkylsulfonylamino steht, |
| $X^1$, $X^2$, $Y^1$ und $Y^2$ | gleich oder verschieden sind und für Wasserstoff, Halogen, Alkyl, Halogenalkyl, Alkoxy, Halogenalkoxy, Alkylthio, Alkylamino, Dialkylamino oder für gegebenen- |

falls substituiertes Phenoxy stehen, und

Z für eine der nachstehenden Gruppierungen steht:

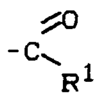 , 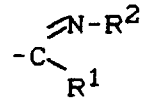 , 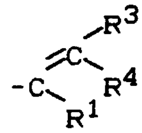 ,

wobei

$R^1$ für Wasserstoff, für gegebenenfalls substituiertes Alkyl oder für gegebenenfalls substituiertes Phenyl steht,

$R^2$ für Wasserstoff, Hydroxy, Amino oder für jeweils gegebenenfalls substituiertes Alkyl, Alkenyl, Alkinyl, Alkoxy, Alkenyloxy, Alkoxycarbonylalkoxy, Dialkylaminocarbonylalkoxy, Alkylamino, Dialkylamino, Alkylcarbonylamino, Alkoxycarbonylamino, Alkylsulfonylamino, Aryl, Aralkyl, Aryloxy, Aralkyloxy, Arylamino, Diarylamino, Aralkylamino, N-Alkyl-N-arylamino, Arylcarbonylamino, Heteroarylamino, Heteroarylcarbonylamino oder Arylsulfonylamino steht,

$R^3$ für Wasserstoff, Halogen, Cyano, Carboxy, Alk-oxycarbonyl, Alkylcarbonylamino oder Dialkoxyphosphoryl steht, und

$R^4$ für Formyl, Cyano, Carboxy, Hydroxymethyl, Carbamoyl oder für jeweils gegebenenfalls substituiertes Alkoxycarbonyl, Cycloalkoxycarbonyl, Alkylthiocarbonyl, Alkylaminocarbonyl, Cycloalkylaminocarbonyl, Dialkylaminocarbonyl, Alkylaminocarbonylalkoxycarbonyl, Dialkylaminocarbonylalkoxycarbonyl, Arylaminocarbonylalkoxycarbonyl, N-Alkyl-N-arylaminocarbonylalkoxycarbonyl, Pyrrolidinylcarbonyl, Piperidinylcarbonyl, Morpholinylcarbonyl, Piperazinylcarbonyl, Aryloxycarbonyl, Aralkyloxycarbonyl, Heterocyclylalkoxycarbonyl, Arylthiocarbonyl, Aralkylthiocarbonyl, Arylaminocarbonyl, Aralkylaminocarbonyl, N-Alkyl-N-arylaminocarbonyl, Arylhydrazinocarbonyl, Alkylhydrazinocarbonyl, Phthalimidoxycarbonyl oder $R^4$ zusammen mit $R^3$ für die Gruppierung $-CO-O-(CH_2)_n-$steht, wobei n für die Zahlen 1 bis 4 steht.

**2.** Bisazinylverbindungen der allgemeinen Formel (I) gemäß Anspruch 1, dadurch gekennzeichnet, daß darin

$A^1$ und $A^2$ gleich oder verschieden sind und für Stickstoff oder eine C-X-Gruppierung stehen, wobei X für Wasserstoff, Halogen, $C_1$-$C_4$-Alkyl oder $C_1$-$C_4$-Alkoxy steht,

$Q^1$ und $Q^2$ gleich oder verschieden sind und für Sauerstoff, Schwefel, NH oder N-$C_1$-$C_4$-Alkyl stehen,

R für Wasserstoff, Amino, Hydroxy, Cyano, Nitro, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino, Di-($C_1$-$C_4$-alkyl)-amino, $C_1$-$C_4$-Alkylcarbonylamino, $C_1$-$C_4$-Alkoxy-carbonylamino oder $C_1$-$C_4$-Alkylsulfonylamino steht,

$X^1$, $X^2$, $Y^1$ und $Y^2$ gleich oder verschieden sind und für Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkylamino, Di-($C_1$-$C_4$-alkyl)-amino oder für Phenoxy stehen, welches gegebenenfalls durch Cyano, Nitro, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio und/oder $C_1$-$C_4$-Alkoxycarbonyl substituiert ist, und

Z für eine der nachstehenden Gruppierungen steht:

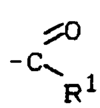 , 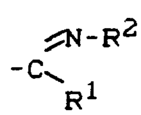 , 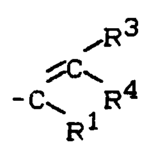 ,

wobei

$R^1$ für Wasserstoff, für gegebenenfalls durch Halogen oder $C_1$-$C_2$-Alkoxy substituiertes $C_1$-$C_4$-Alkyl oder für gegebenenfalls durch Halogen, $C_1$-$C_4$-Alkyl und/oder $C_1$-$C_4$-Alkoxy substituiertes Phenyl steht,

$R^2$ für Wasserstoff, Hydroxy, Amino oder für jeweils gegebenenfalls durch Halogen substituiertes $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxycarbonyl-$C_1$-$C_2$-alkoxy, Di-($C_1$-$C_4$-alkyl)-aminocarbonyl-$C_1$-$C_2$-alkoxy, $C_1$-$C_6$-Alkylamino, Di-($C_1$-$C_2$-alkyl)-amino, $C_1$-$C_6$-Alkylcarbonylamino, $C_1$-$C_6$-Alkoxycarbonylamino, $C_1$-$C_6$-Alkylsulfonylamino, oder für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxycarbonyl und/oder Di-($C_1$-$C_2$-alkyl)-amino substituiertes Phenyl, Phenyl-$C_1$-$C_4$-alkyl, Phenoxy, Phenyl-$C_1$-$C_4$-alkoxy, Phenylamino, Diphenylamino, Phenyl-$C_1$-$C_4$-alkylamino, N-($C_1$-$C_4$-alkyl)-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^3$ für Wasserstoff, Halogen, Cyano, Carboxy, $C_1$- $C_6$-Alkoxycarbonyl, $C_1$-$C_6$-Alkylcarbonylamino oder Di-($C_1$-$C_4$-alkoxy)-phosphoryl steht und

$R^4$ für Formyl, Cyano, Carboxy, Hydroxymethyl, Carbamoyl oder für jeweils gegebenenfalls durch Halogen, Carboxy oder $C_1$-$C_4$-Alkoxycarbonyl substituiertes $C_1$-$C_6$-Alkoxycarbonyl, $C_5$-$C_6$-Cycloalkyloxycarbonyl, $C_1$-$C_6$-Alkylthiocarbonyl, $C_1$-$C_6$-Alkylaminocarbonyl oder $C_5$-$C_6$-Cycloalkylaminocarbonyl, für Di-($C_1$-$C_2$-alkyl)-aminocarbonyl, für $C_1$-$C_4$-Alkylamino-carbonyl-$C_1$-$C_4$-alkoxycarbonyl, für Di-($C_1$-$C_2$-alkyl)-aminocarbonyl-$C_1$-$C_4$-alkoxycarbonyl, für Phenylaminocarbonyl-$C_1$-$C_4$-alkoxycarbonyl, für N-Methyl-N-phenylaminocarbonyl-$C_1$-$C_4$-alkoxycarbonyl, für jeweils gegebenenfalls durch Methyl und/oder Ethyl substituiertes Pyrrolidinylcarbonyl, Piperidinyl-carbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxycarbonyl und/oder Di-($C_1$-$C_2$-alkyl)-amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthiocarbonyl, Phenylaminocarbonyl, Phenyl-$C_1$-$C_4$-alkylaminocarbonyl, N-($C_1$-$C_4$-Alkyl)-N-phenylaminocarbonyl oder Phenylhydrazinocarbonyl, $C_1$-$C_4$-Alkylhydrazinocarbonyl, für Phthalimidoxycarbonyl oder zusammen mit $R^3$ für die Gruppierung $-CO-O-(CH_2)_n-$ steht, wobei

n für die Zahlen 1 bis 4, insbesondere 2 oder 3 steht.

**3.** Verfahren zur Herstellung von Bisazinylverbindungen der Formel (I) gemäß Anspruch 1, dadurch gekennzeichnet, daß man

(a) Hydroxyverbindungen der allgemeinen Formel (II),

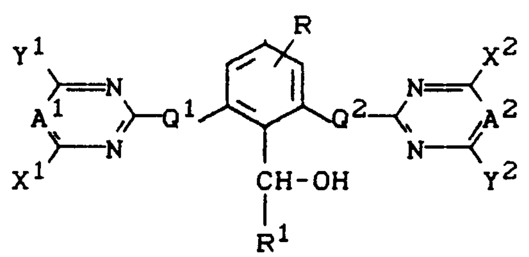

(II)

in welcher

$A^1$, $A^2$, $Q^1$, $Q^2$, R, $R^1$, $X^1$, $X^2$, $Y^1$ und $Y^2$ die in Anspruch 1 angegebene Bedeutung haben,

mit Oxidationsmitteln ("Dehydrierungsmitteln") gegebenenfalls in Gegenwart von Verdünnungsmitteln umsetzt, oder daß man

(b) Benzolderivate der allgemeinen Formel (III),

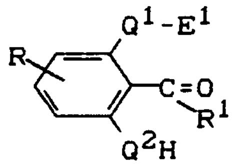

(III)

in welcher

$Q^1$, $Q^2$, R und $R^1$ die oben angegebene Bedeutung haben und

$E^1$ für Wasserstoff oder die Gruppierung

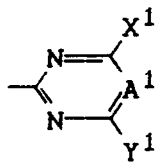

steht, wobei

$A^1$, $X^1$ und $Y^1$ die oben angegebene Bedeutung haben,

mit reaktionsfähigen Azinen der allgemeinen Formel (IVa)

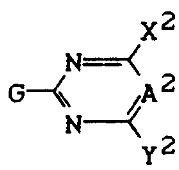

(IVa)

und gegebenenfalls auch mit reaktionsfähigen Azinen der allgemeinen Formel (IVb),

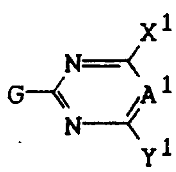

(IVb)

wobei

$A^1$, $A^2$, $X^1$, $X^2$, $Y^1$ und $Y^2$ die oben angegebene Bedeutung haben und

G für eine nucleophile Abgangsgruppe steht,

gegebenenfalls in Gegenwart eines Säureakzeptors und gegebenenfalls in Gegenwart eines Verdünnungsmittels umsetzt, oder daß man

(c) Carbonylverbindungen der allgemeinen Formel (Ia),

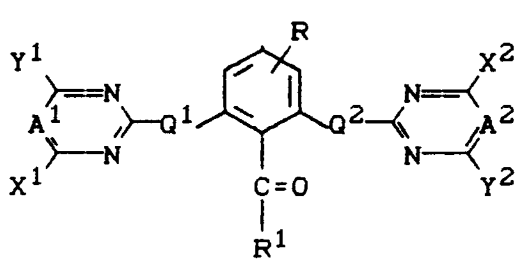

(Ia)

in welcher
$A^1$, $A^2$, $Q^1$, $Q^2$, R, $R^1$, $X^1$, $X^2$, $Y^1$ und $Y^2$ die oben angegebene Bedeutung haben,
mit Aminoverbindungen der allgemeinen Formel (V),

$H_2N\text{-}R^2$ (V)

in welcher
$R^2$ die oben angegebene Bedeutung hat,
oder mit Methylenverbindungen der allgemeinen Formel (VI),

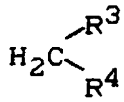

(VI)

in welcher
$R^3$ und $R^4$ die oben angegebene Bedeutung haben,
gegebenenfalls in Gegenwart eines Reaktionshilfsmittels und gegebenenfalls in Gegenwart eines Verdünnungsmittels umsetzt und gegebenenfalls die so erhaltenen Produkte der Formel (I) anschließend nach üblichen Methoden in andere Derivate gemäß der Definition der Verbindungen der Formel (I) umwandelt.

**4.** Herbizide Mittel, gekennzeichnet durch einen Gehalt an mindestens einer Bisazinylverbindung der Formel (I) gemäß Anspruch 1.

**5.** Verfahren zur Bekämpfung von unerwünschten Pflanzen, dadurch gekennzeichnet, daß man eine Bisazinylverbindung der Formel (I) gemäß Anspruch 1 auf die unerwünschten Pflanzen und/oder ihren Lebensraum einwirken läßt`

**6.** Verwendung von Bisazinylverbindungen der Formel (I) gemäß Anspruch 1 zur Bekämpfung von unerwünschten Pflanzen.

**7.** Verfahren zur Herstellung von herbiziden Mitteln, dadurch gekennzeichnet, daß man eine Bisazinylverbindung der Formel (I) gemäß Anspruch 1 mit Streckmitteln und/oder oberflächenaktiven Substanzen vermischt.

**8.** Hydroxyverbindungen der allgemeinen Formel (II),

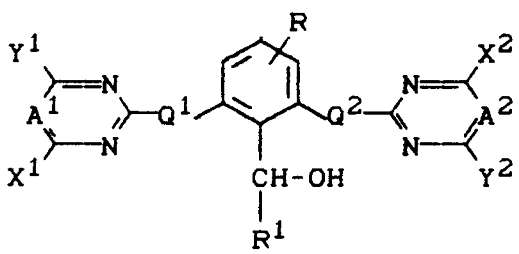

(II)

in welcher

| | |
|---|---|
| $A^1$ und $A^2$ | gleich oder verschieden sind und für Stickstoff oder eine C-X-Gruppierung stehen, wobei X für Wasserstoff, Halogen, Alkyl oder Alkoxy steht, |
| $Q^1$ und $Q^2$ | gleich oder verschieden sind und für Sauerstoff, Schwefel, NH oder N-Alkyl stehen, |
| R | für Wasserstoff, Amino, Hydroxy, Cyano, Nitro, Halogen, Alkyl, Halogenalkyl, Alkoxy, Halogenalkoxy, Alkylthio, Halogenalkylthio, Alkylamino, Dialkylamino, Alkylcarbonylamino, Alkoxycarbonylamino oder Alkylsulfonylamino steht, |
| $X^1$, $X^2$, $Y^1$ und $Y^2$ | gleich oder verschieden sind und für Wasserstoff, Halogen, Alkyl, Halogenalkyl, Alkoxy, Halogenalkoxy, Alkylthio, Alkylamino, Dialkylamino oder für gegebenen- |

falls substituiertes Phenoxy stehen, und

$R^1$ für Wasserstoff, für gegebenenfalls substituiertes Alkyl oder für gegebenenfalls substituiertes Phenyl steht,

**9.** Verbindungen der Formel (IIIa),

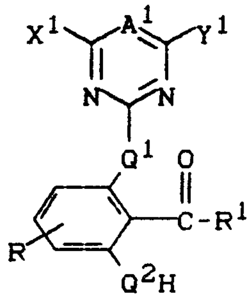

(IIIa)

in welcher

$A^1$ für Stickstoff oder eine C-X-Gruppierung steht, wobei X für Wasserstoff, Halogen, Alkyl oder Alkoxy steht,

$Q^1$ und $Q^2$ gleich oder verschieden sind und für Sauerstoff, Schwefel, NH oder N-Alkyl stehen,

R für Wasserstoff, Amino, Hydroxy, Cyano, Nitro, Halogen, Alkyl, Halogenalkyl, Alkoxy, Halogenalkoxy, Alkylthio, Halogenalkylthio, Alkylamino, Dialkylamino, Alkylcarbonylamino, Alkoxycarbonylamino oder Alkylsulfonylamino steht,

$X^1$ und $Y^1$ gleich oder verschieden sind und für Wasserstoff, Halogen, Alkyl, Halogenalkyl, Alkoxy, Halogenalkoxy, Alkylthio, Alkylamino, Dialkylamino oder für gegebenenfalls substituiertes Phenoxy stehen, und

$R^1$ für Wasserstoff, für gegebenenfalls substituiertes Alkyl oder für gegebenenfalls substituiertes Phenyl steht.

**10.** Halogenalkylverbindungen der allgemeinen Formel (VIII),

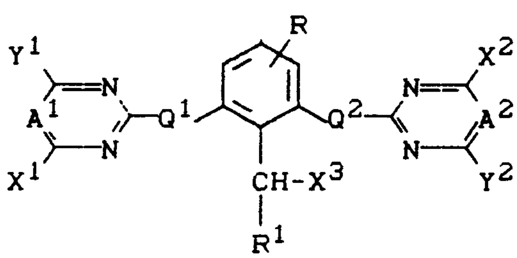

(VIII)

in welcher

$A^1$ und $A^2$ gleich oder verschieden sind und für Stickstoff oder eine C-X-Gruppierung stehen, wobei X für Wasserstoff, Halogen, Alkyl oder Alkoxy steht,

$Q^1$ und $Q^2$ gleich oder verschieden sind und für Sauerstoff, Schwefel, NH oder N-Alkyl stehen,

R für Wasserstoff, Amino, Hydroxy, Cyano, Nitro, Halogen, Alkyl, Halogenalkyl, Alkoxy, Halogenalkoxy, Alkylthio, Halogenalkylthio, Alkylamino, Dialkylamino, Alkylcarbonylamino, Alkoxycarbonylamino oder Alkylsulfonylamino steht,

$X^1$, $X^2$, $Y^1$ und $Y^2$ gleich oder verschieden sind und für Wasserstoff, Halogen, Alkyl, Halogenalkyl, Alkoxy, Halogenalkoxy, Alkylthio, Alkylamino, Dialkylamino oder für gegebenenfalls substituiertes Phenoxy stehen,

$R^1$ für Wasserstoff, für gegebenenfalls substituiertes Alkyl oder für gegebenenfalls substituiertes Phenyl steht, und

$X^3$ für Chlor oder Brom steht.

Europäisches Patentamt

# EUROPÄISCHER RECHERCHENBERICHT

Nummer der Anmeldung

EP 91115182.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁴) |
|---|---|---|---|
| D,A | EP - A2 - 0 321 846 (KUMIAI, IMARA) * Ansprüche 1,10,11,12,13, 14 * | 1,3,4, 5,6,7 | C 07 D 239/60<br>C 07 D 239/28<br>C 07 D 239/32<br>C 07 D 239/46<br>C 07 D 251/26<br>C 07 D 251/30<br>C 07 D 251/38<br>A 01 N 43/54<br>A 01 N 43/70 |
| D,A | EP - A2 - 0 336 494 (SHELL) * Ansprüche 1,9,10 * | 4,5,6, 7,9 | |
| A | GB - A - 1 585 950 (ICI) * Ansprüche 1,10,12,14; Verbindung Nr. 43 * | 1,4,5, 6,7,9 | |

RECHERCHIERTE SACHGEBIETE (Int. Cl.⁴)

C 07 D 239/00
C 07 D 251/00
A 01 N 43/00

Der vorliegende Recherchenbericht wurde für alle Patentansprüche erstellt.

| Recherchenort | Abschlußdatum der Recherche | Prüfer |
|---|---|---|
| WIEN | 08-11-1991 | LUX |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument